# NEW EUROPEAN PATENT SPECIFICATION

(11) **EP 0 968 934 B2**
(45) Date of publication and mention of the opposition decision: **05.09.2007**
(45) Mention of the grant of the patent: 12.02.2003
(21) Application number: 99304732.3
(22) Date of filing: 17.06.1999
(51) Int. Cl.: B65D 83/08, B65D 75/58

(54) **Package containing stacked sheet products**
Gestapelte Blattprodukte enthaltende Verpackung
Emballage contenant une pile de produits en feuille

(30) Priority: 17.06.1998 JP 16960698
(43) Date of publication of application: 05.01.2000
(73) Proprietor: UNI-CHARM CORPORATION, Kawanoe-shi Ehime-ken (JP)
(72) Inventor: Bando, Takeshi, Technical Center, Mitoyo-gun, Kanagawa-ken 769-1602 (JP); Tanaka, Yoshikazu, Technical Center, Mitoyo-gun, Kanagawa-ken 769-1602 (JP); Naruse, Motokazu, Technical Center, Mitoyo-gun, Kanagawa-ken 769-1602 (JP)
(74) Representative: Parry, Christopher Stephen

(56) References cited:
- EP-A- 0 307 924
- EP-B- 0 420 301
- DE-U- 9 208 932
- US-A- 3 819 043
- US-A- 4 420 080
- US-A- 4 863 064
- US-A- 5 582 342

## Description

The present invention relates to a package having a plurality of sheet products stacked therein, which has an opening of a small opening area, from which the sheet products can be picked up easily.

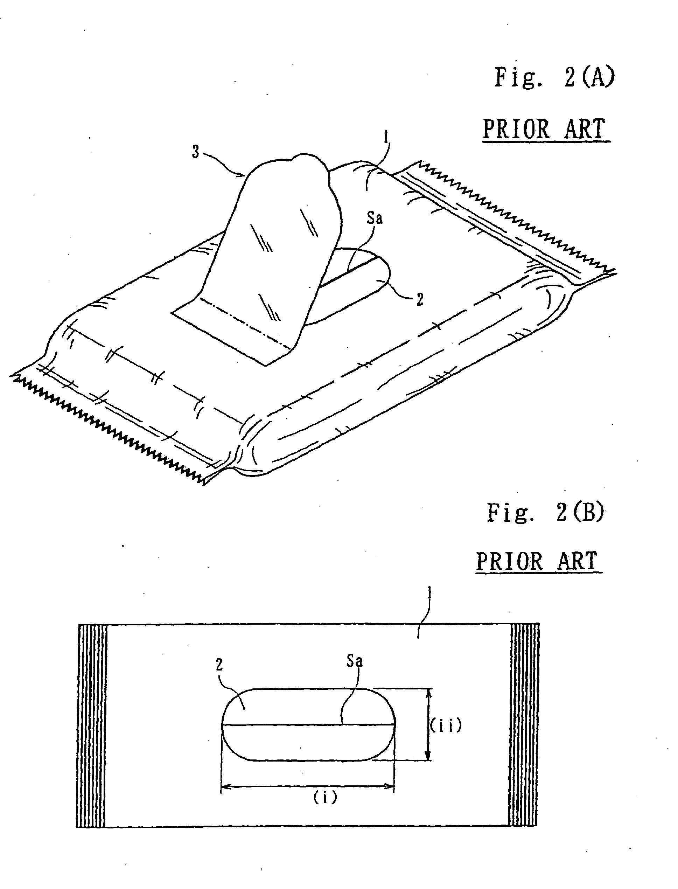

**Fig. 2(A)** is a perspective view showing a conventional handy package from which stacked sheet products such as wet tissue papers can be picked up one after another. The main body of the conventional package is composed of an envelope **1** formed from, for example, a piece of laminated film, and the envelope **1** houses the stacked wet tissue papers therein.

**Fig. 2(B)** is a plan view showing the package in **Fig. 2(A),** with a lid **3** removed. An opening **2** is formed on the surface of the envelope **1**, and an edge portion **Sa** of the uppermost one of the wet tissue papers appears therein. By pinching and pulling the edge portion **Sa** with two fingers through the opening **2**, the wet tissue papers housed in the envelope **1** are picked up one after another. The opening **2** is formed in an oval or elliptic shape, with the major axis **(i)** parallel to the edge portion **Sa** and with the minor axis **(ii)** perpendicular to the edge portion **Sa.** In such a handy package, it should be noted that the opening area of the opening **2** is required to be as small as possible, for preventing the wet tissue paper from drying in use. In the package shown in **Figs. 2(A)** and **2(B)**, because the dimensions of the opening **2** are much,larger in the direction parallel to the edge portion **Sa** than in the direction perpendicular to the edge portion **Sa,** it enables picking the wet tissue papers without providing an excessive resistance between the wet tissue papers and the edge of the opening 2. The opening 2 is usually closed with the lid 3 for preventing the wet tissue paper from drying, and opened by pulling a tab portion of the lid 3 only in use. The lid 3 has an adhesive layer such as of pressure adhesive on the side opposite to the envelope 1, and the adhesive layer enables the lid 3 to removing from and attaching to the periphery of the opening 2 repeatedly. In such a handy package, it should be also noted that the lid 3 is provided to be pulled in the lengthwise direction of the envelope 1 as shown in Figs. 2(A), because the overall size of the envelope 1 is so small.

However, the opening 2 in the conventional handy package shown in Figs. 2(A) and 2(B) has such a problem that because the dimension of the opening 2 in the direction perpendicular to the edge portion Sa (i.e. the minor axis (ii)) is too small, it is difficult to separate two fingers from each other sufficiently within the opening 2, whereby the edge portion Sa is difficult to pinch with two fingers.

Further, because the opening 2 elongates much in the lengthwise direction of the envelope 1, the lid 3 also elongates much in the same direction. Therefore, the lid 3 has to be pulled a relatively long distance to open the opening 2, and there is a possibility that the opening 2 is not sealed completed, because the lid 3 or the envelope 1 is wrinkled when the lid 3 is attached to the periphery of the opening 2.

US-A-3819043 discloses a package having a plurality of sheet products stacked to each other therein, the package comprising an envelope for housing the stacked sheet products and having a lengthwise dimension and a widthwise dimension, the widthwise dimension being shorter than the lengthwise dimension, the envelope having an opening in which an edge portion of the uppermost one of the stacked sheet products extending in the lengthwise direction appears and through which it is pinched by fingers in use, wherein the dimensions of the opening are larger in the widthwise direction than in the lengthwise direction; the package further comprising a lid attached to the envelope adjacent the opening for opening and closing it, the lid having a base portion and a cover portion. The base portion of the lid is attached to the package alongside a side of the opening extending in the lengthwise direction. The lid is flexible so that it automatically returns to the closed position.

Document US-A-4 420 080, discloses a resealable dispenser-container with a transversal opening covered by an adhesive lid.

The present invention is to solve the conventional problems as mentioned above, and an object of the present invention is to provide a package having a plurality of sheet products stacked therein, which has an opening of a small opening area, from which the sheet products can be picked up easily.

It is another object of the present invention to provide a package having a plurality of sheet products stacked therein, which is provided with a lid which can open an opening easily and can close the opening securely.

The present invention is to provide a package according to claim 1.

Further, the opening is preferably formed in an oval or elliptic shape. The dimensions of the opening are preferably 40±3mm and 30±3mm.

Moreover, it is preferred that the envelope is formed from a laminated film of a polyethylene terephthalate film, an aluminum and a sealant film, and the lid is formed from a biaxially oriented polypropylene film.

Embodiments of the invention are described below with reference to the accompanying drawings, in which:
Fig. 1(A) is a perspective view showing an embodiment of a package according to the present invention, Fig. 1(B) is a plan view of the package shown in Fig. 1(A) and Fig. 1(C) is a cross-sectional view of the package taken along the line **C**-**C** shown in **Fig. 1(B)**; and
**Fig. 2(A)** is a perspective view showing a conventional package, **Fig. 2(B)** is a plan view of the package shown in **Fig. 2(A).**

The invention is described in more detail with reference to the accompanying drawings.

As used herein, "comprises" and all its grammatical forms specifies the presence of stated features, integers, steps or components, but does not preclude the presence or addition of one or more other features, integers, steps, components or groups thereof.

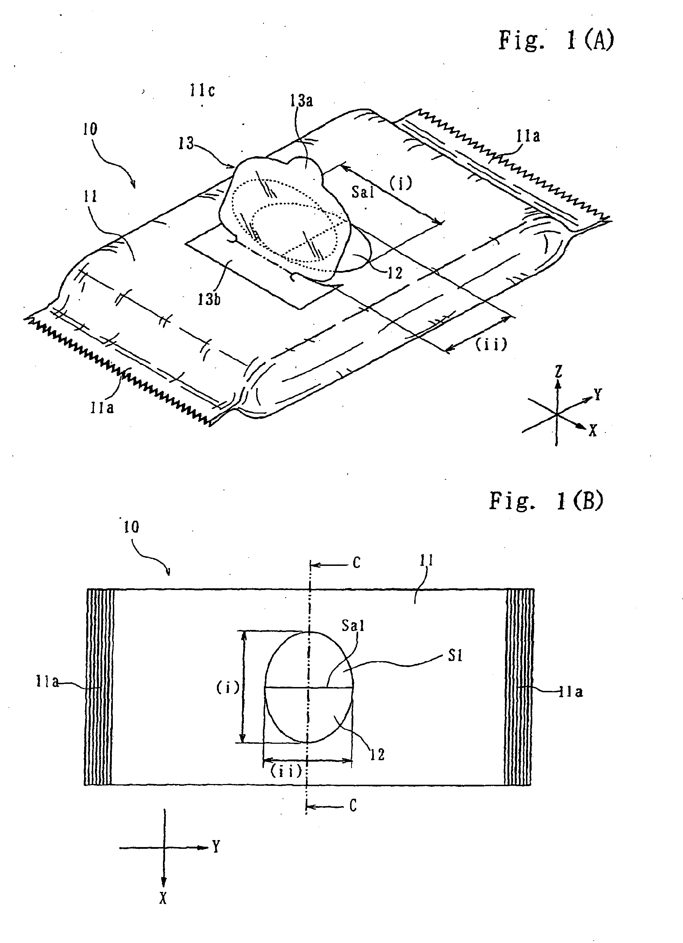

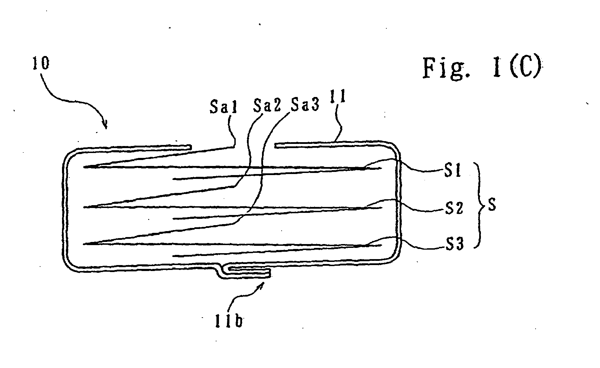

**Fig. 1(A)** is a perspective view showing an embodiment of a package according to the present invention, **Fig. 1(B)** is a plan view of the package shown in **Fig. 1(A)** with a lid removed, and **Fig. 1(C)** is a cross-sectional view of the package with the lid removed, taken along the line **C**-**C** shown in **Fig. 1(B)**.

A package **10** shown in **Figs. 1(A)** to **1(C)** is made handy, for example, to have the dimensions of 15cm, 8cm and 3cm, in length (Y direction), width (X direction) and height (Z direction), respectively. The package **10** comprises an envelope **11**, a plurality of wet tissue papers **S** impregnated with water or chemicals and a lid **13**. The envelope **11** is formed from a piece of film such that the wet tissue papers **S** are enveloped by folding the film and then sealed therein by forming a lengthwise seal portion **11b** and widthwise seal portions **11a**. The envelope **11** has an opening **12** for pinching and pulling the wet tissue papers **S** one after another with two fingers. The lid **13** is for closing or opening the opening **12** in use.

The film forming the envelope **11** is preferably a laminated film, which is formed by laminating a PET (polyethylene terephthalate) film, an aluminum foil and a sealant film such as biaxially oriented polypropylene film having a heat-sealing property, in order from outside to inside of the envelope **11**, and then joining them to one another. Alternatively, the laminated film may be formed by laminating and joining a sealant film having a heat-sealing property inside of a PET film deposited with aluminum, silica or alumina thereon. With the sealant film facing inside of the envelope **11** (i.e., facing the wet tissue papers **S),** as described above, the lengthwise seal portion **11b** and the widthwise seal portions **11a** can be formed by heat-sealing (i.e., by heating the sealant film under pressure). On the surfaces of the envelope **11**, for example, figures, symbols, letters such as trade name and operating instruction, etc., are printed.

**Fig. 1(C)** schematically shows a stacked state of the wet tissue papers **S** in the envelope **11,** by using three pieces of wet tissue papers **S1, S2** and **S3**. The number of the wet tissue papers **S** to be housed in the envelope **11** is optional. Generally, in such a handy package, the number of wet tissue papers to be housed is about 30 to 40 pieces. The wet tissue papers **S1, S2** and **S3** are folded respectively, and then stacked to one another such that edge portions **Sa1, Sa2** and **Sa3,** which linearly extending in the lengthwise direction (Y direction) of the envelope **11**, conform to the substantial center of the opening **12** as shown in **Fig. 1(C).**

The lid **13** shown in **Fig. 1(A)** has a tab portion **13a** at one end thereof in the lengthwise direction (Y direction) of the envelope **11** and a base portion **13b** at the other end thereof in the lengthwise direction (Y direction) of the envelope **11**. The main body of the lid **13** is formed from a biaxially oriented polypropylene film. The lid 13 has an adhesive layer, such as of pressure sensitive adhesive, provided on the entire surface opposite to the envelope **11**, except for the tab portion **13a**. The pressure sensitive adhesive mainly contains an acrylic type adhesive, a polyvinyl chloride composition containing plasticizer, or a graftmer such as graft copolymer formed by graft polymerizing ethylene-vinyl acetate copolymer and vinyl chloride monomer. With such pressure sensitive adhesive, the lid **13** can be removed from and attached to the surface of the envelope **11** repeatedly. When the wet tissue papers **S** are picked up from the opening **12** in use, the lid **13** is removed from the periphery of the opening **12** by pulling the tab portions **13a,** except that the base portion **13b** remains still as shown in **Fig. 1(A)**. Then, after picking up the wet tissue papers **S,** the lid **13** is attached to the periphery of the opening **12** to close the opening **12.**

Incidentally, the film to form the envelope **11** is formed with notches or perforations arranged in the outline of the opening **12** in advance of forming the envelope **11**. In the production process of the package **10,** the lid **13** is attached to the surface of the envelope **11** with the above-described adhesive layer, so as to cover the notches or perforations. When the package **10** is used for the first time, the lid **13** is pulled up while imparting the adhesive power to a portion **11c** which is surrounded by the notches or perforations. Thus, the portion **11c** is separated from the envelope **11** along the notches or perforations to open the opening **12** in the envelope **11.** Thereafter, the portion **11c** remains attached to the surface of the lid **13** as shown in **Fig. 1(A).** Because the envelope **11** is sealed almost completely until the opening **12** is opened by pulling up the lid **13** for the first time, the wet tissue papers **S** impregnated with water or chemicals can be prevented from drying. Also, because the aluminum foil in the laminated film is impermeable to water and oxygen and has an excellent air tightness, it also contributes to the prevention of the drying of the wet tissue papers **S.**

The wet tissue papers S are picked up one by one from the opening **12**. As shown in **Fig. 1(B)**, the uppermost wet tissue paper **S1** is exposed from the opening **12,** with the edge portion **Sa1** conforming to the substantial center of the opening **12.** The wet tissue paper **S1** is picked up by pinching and pulling the edge portion **Sa1** with two fingers (e.g. thumb and forefinger) through the opening **12.** In this embodiment, in order to make the opening **12** as small as possible for preventing the wet tissue papers S from drying, the opening **12** is formed in an oval or elliptic shape, with its major axis **(i)** perpendicular to the edge portion **Sa1** and with the minor axis **(ii)** parallel to (identical to) the edge portion **Sa1**. That is, the dimensions of the opening **12** are larger in the direction perpendicular to the edge portion **Sa1** than in the direction parallel to the edge portion **Sa1** (i.e. larger in X direction than in Y direction). That is because the edge portion **Sa1** is to be pinched with two fingers from the direction perpendicular to the edge portion **Sa1**.

It is preferred that the major axis **(i)** of the opening **12** is 40±3mm. Within this range, two fingers, which are inserted into the opening **12,** can be separated from each other sufficiently within the opening **12** in the widthwise direction of the package **10**. However, if it is above the upper limit, the opening area of the opening **12** becomes too large insignificantly, so that the water or chemicals contained in the wet tissue papers S is easily dried.

It is preferred that the minor axis **(ii)** of the opening **12** is 30±3mm. Within this range, the thumb can be inserted into the opening **12** allowing a margin. However, if it is above the upper limit, the opening area of the opening **12** becomes too large insignificantly, so that the water or chemicals contained in the wet tissue papers **S** is easily dried.

It should be noted that the shape of the opening **12** is not limited to an oval or elliptic shape, it may be formed in any shape such as rectangular shape so long as the dimensions of the opening **12** are larger in the direction perpendicular to the edge portion **Sa1** than in the direction parallel to the edge portion **Sa1**. It is preferred that the dimensions -length and width- of the opening **12** in a rectangular shape are respectively 40±3mm and 30±3mm.

The lid **13** is provided to the envelope **11** such that the lid **13** is pulled along the lengthwise direction of the envelope **11,** because the envelope **11** does not have a sufficient dimension for the lid **13** in the widthwise direction. In the embodiment of the present invention, the minor axis **(ii)** of the opening **12** extends along the lengthwise direction of the envelope **11**. Thus, the dimension of the lid **13** in the lengthwise direction of the envelope **11** can be made smaller, as compared with the conventional package shown in **Fig. 2(A).** Providing the lid **13** in such way as mentioned-above, the opening **12** can be easily opened by pulling the tab portion **13a** of the lid **13** in the lengthwise direction of the envelope **11** a relatively short distance. Further, owing to the small dimension of the lid **13** in the lengthwise direction of the envelope **11**, the envelope **11** or the lid **13** is prevented from being wrinkled, when the opening **12** is closed with the lid **13.** Thus, the opening **12** can be completely sealed with the lid **13** for preventing the wet tissue papers **S** from drying.

While in the foregoing specification the present invention has been described in relation to the preferred embodiment and many details have been set forth for purpose of illustration, it should be apparent to those skilled in the art that the present invention is susceptible to additional embodiments, and that certain details described herein can be varied considerably without departing from the basic principles of the present invention.

For example, the opening **12** may be opened in the production process instead of opening the opening **12** by separating the portion **11c** from the envelope **11** by pulling the lid 13 in use.

The envelope 11 may be formed from paper or may be molded of plastic material in a box shape. The material of the lid 13 can be changed in accordance with the material of the envelope 11.

## Claims

1. A package (10) having a plurality of sheet products stacked to each other therein in a non-interleaved manner, wherein the sheet products are wet tissue papers the package comprising a rectangular envelope (11) for housing the stacked sheet products (5) and having a lengthwise dimension and a widthwise dimension, the widthwise dimension being shorter than the lengthwise dimension, the envelope having an opening (12) for pinching and pulling one after another the stacked sheet products, wherein each sheet product extending in the lengthwise direction (Y) in turn appears as an uppermost one of the stacked sheet products with each respective edge portion (Sa1, Sa2, Sa3, ...) linearly extending in the lengthwise direction (Y) and conforming to the substantial centre of the opening, appearing in turn through the opening and through which opening it is pinched by fingers in use,
wherein the dimensions of the opening are larger in the widthwise direction (X) than in the lengthwise direction (Y);
the package further comprising a lid (13) attached to the envelope adjacent the opening for opening and closing it, the lid having a tab portion, a base portion (13b) on opposite sides in the direction parallel to the edge portion and a cover portion therebetween ;
the lid having an adhesive layer thereon enabling the lid to be removed from and attached to the surface of the envelope repeatedly
wherein the base portion (13b) of the lid is attached to the envelope alongside a side of the opening (12) extending in the widthwise direction (X), the base portion being arranged to remain attached when the cover portion is removed from the periphery of the opening.

2. The package of any preceding claim, wherein:
the opening (12) is formed in an oval or elliptic shape.

3. The package of any preceding claim, wherein:
the dimensions of the opening (12) are 40±3mm and 30±3mm.

4. The package of any preceding claim, wherein:
the adhesive layer is of a pressure sensitive adhesive.

5. The package of any preceding claim, wherein:
the envelope (10) is formed from a laminated film of a polyethylene terephthalate film, an aluminium foil and a sealant film, and the lid is formed from a biaxially oriented polypropylene film.

## Patentansprüche

1. Packung (10) mit einer Mehrheit von Blattprodukten, die darin auf eine nicht verschachtelte Weise aneinander gestapelt sind, wobei die Blattprodukte nasses Tissue-Papier sind, die Packung eine rechteckige Hülle (11) zur Unterbringung der gestapelten Blattprodukte (5) umfasst und eine Längsdimension und eine Breitendimension aufweist, wobei die Breitendimension kürzer als die Längsdimension ist, wobei die Hülle eine Öffnung (12) zum Kneifen und Herausziehen der gestapelten Blattprodukte eins nach dem anderen aufweist, wobei jedes Blattprodukt, das sich in der Längsrichtung (Y) erstreckt, der Reihe nach als das Oberste der gestapelten Blattprodukte erscheint, wobei sich der jeweiligen Kantenteil (Sal, Sa2, Sa3...) linear in der Längsrichtung (Y) erstreckt und entsprechend der wesentlich mittigen Öffnung der Reihe nach durch die Öffnung erscheint und durch welche Öffnung der Kantenteil in Gebrauch mit den Fingern gekneift wird,
wobei die Dimensionen der Öffnung größer in der Breitenrichtung (X), als in der Längsrichtung (Y) sind;
die Packung weiter einen Deckel (13) umfasst, der an der Hülle angrenzend an die Öffnung zum Öffnung und Schließen befestigt ist, wobei der Deckel einen Laschenteil, einen Basisteil (13b) an den gegenüberliegenden Seiten in der Richtung parallel zum Kantenteil und einen Abdeckteil dazwischen umfasst;
der Deckel eine Klebschicht daran hat, die ermöglicht den Deckel von der Oberfläche der Hülle wiederholt zu entfernen und wieder an diese zu befestigen;
wobei der Basisteil (13b) des Deckels an die Hülle entlang einer Seite der Öffnung (12) befestigt ist, die sich in der Breitenrichtung (X) erstreckt, wobei der Basisteil eingerichtet ist befestigt zu bleiben, wenn der Abdeckteil von der Peripherie der Öffnung entfernt wird.

2. Packung nach einem beliebigen vorhergehenden Anspruch, wobei:
Die Öffnung (12) in einer ovalen oder elliptischen Form gebildet ist;

3. Packung nach einem beliebigen vorhergehenden Anspruch, wobei:
Die Dimensionen der Öffnung (12) 40±3 mm und 30±3 mm sind.

4. Packung nach einem beliebigen vorhergehenden Anspruch, wobei:
Die Kleberschicht ein Haftklebstoff ist.

5. Packung nach einem beliebigen vorhergehenden Anspruch, wobei:
Die Hülle (10) aus einer laminierten Folie einer Polyethylenterephthalatfolie, einer Aluminiumfolie und einer Dichtmittelfolie gebildet ist und der Deckel aus einer biaxial orientierten Polypropylenfolie gebildet ist.

## Revendications

1. Emballage (10) comportant à l'intérieur une pluralité de produits en feuilles empilés l'un sur l'autre d'une façon non-intercalée, dans lequel les produits en feuilles sont des papiers de soie humides, l'emballage comprenant une enveloppe rectangulaire (11) destinée à loger les produits en feuilles empilés (5) et présentant une dimension dans le sens de la longueur et une dimension dans le sens de la largeur, la dimension dans le sens de la largeur étant inférieure à la dimension dans le sens de la longueur, l'enveloppe présentant une ouverture (12) pour pincer et tirer l'un après l'autre les produits en feuilles empilés, dans lequel chaque produit en feuille s'étendant dans le sens de la longueur (Y) apparaît à son tour comme celui qui est le plus élevé parmi les produits en feuilles empilés, avec chaque partie de bord respective (Sa1, Sa2, Sa3,...), s'étendant linéairement dans le sens de la longueur (Y) et se conformant essentiellement au centre de l'ouverture, apparaissant à son tour à travers l'ouverture, ouverture au travers de laquelle il est pincé par des doigts en utilisation ;
dans lequel les dimensions de l'ouverture sont supérieures dans le sens de la largeur (X) à celles dans le sens de la longueur (Y) ;
l'emballage comprenant de plus une coiffe (13) fixée à l'enveloppe, adjacente à l'ouverture afin de la fermer et de l'ouvrir, la coiffe comportant une partie de languette, une partie de base (13b) au niveau de l'autre extrémité de celle-ci dans une direction parallèle à la portion de bord, et une partie de couvercle entre celles-ci ;
la coiffe comportant sur elle une couche adhésive permettant de fixer la coiffe à la surface de l'enveloppe et de l'enlever de manière répétée,
dans lequel la partie de base (13b) de la coiffe est fixée à l'enveloppe le long d'un côté de l'ouverture (12) qui s'étend dans le sens de la largeur (X), la partie de base étant agencée pour rester attachée lorsque la partie de couvercle est enlevée de la périphérie de l'ouverture.

2. Emballage la revendication 1, dans lequel :
l'ouverture (12) est de forme ovale ou elliptique.

3. Emballage selon l'une quelconque des revendications précédentes, dans lequel :
les dimensions de l'ouverture (12) sont de 40 ±3 mm et de 30 ±3 mm.

4. Emballage selon l'une quelconque des revendications précédentes, dans lequel :
la couche adhésive est un adhésif sensible à la pression.

5. Emballage selon l'une quelconque des revendications précédentes, dans lequel :
l'enveloppe (10) est formée d'un film laminé à partir d'un film de polyéthylène térephtalate, d'un film d'aluminium et d'un film d'étanchéité, et la coiffe est formée d'un film de polypropylène bi-orienté.
